# FASCICULE DE BREVET EUROPEEN

(11) **EP 3 463 607 B1**
(45) Date de publication et mention de la délivrance du brevet: **13.04.2022**
(21) Numéro de dépôt: 17721405.3
(22) Date de dépôt: 05.05.2017
(51) Int. Cl.: B01D 15/18, B01D 15/22, B01D 15/14, G01N 30/44, G01N 30/46, G01N 30/60, B01D 1/02, B01D 1/14, C10G 25/00

(54) **NOUVEAU SYSTÈME DE DISTRIBUTION OU DE COLLECTE PÉRIPHÉRIQUE POUR UN PROCÉDÉ DE SÉPARATION EN LIT MOBILE SIMULÉ UTILISANT N-COLONNES EN SÉRIE**
NEUARTIGES PERIPHERES VERTEILUNGS- ODER SAMMELSYSTEM FÜR EIN SIMULIERTES WANDERBETTTRENNVERFAHREN MIT N-SÄULEN IN REIHE
NOVEL PERIPHERAL DISTRIBUTION OR COLLECTION SYSTEM FOR A SIMULATED MOVING BED SEPARATION METHOD USING N-COLUMNS IN SERIES

(30) Priorité: 30.05.2016 FR 1654864
(43) Date de publication de la demande: 10.04.2019
(73) Titulaire: IFP Energies nouvelles, 92852 Rueil-Malmaison (FR)
(72) Inventeur: AUGIER, Frederic, 69360 Saint-Symphorien-d'Ozon (FR); ROYON-LEBEAUD, Aude, 69007 Lyon (FR); LEINEKUGEL LE COCQ, Damien, 69007 Lyon (FR); VONNER, Alexandre, 69780 Mions (FR)
(86) Numéro de dépôt international: PCT/EP2017/060835
(87) Numéro de publication internationale: WO 2017/207217

(56) Documents cités:
- EP-A1- 0 842 687
- WO-A1-2013/089774
- FR-A1- 2 740 052
- FR-A1- 2 794 663

## Description

### CONTEXTE DE L'INVENTION

L'invention concerne un nouveau dispositif de distribution et de collecte de fluides au sein d'un système à N-colonnes en série mettant en œuvre un écoulement desdits fluides dans un milieu de particules solides, appelé milieu granulaire.

On appelle système à N-colonnes en série un enchainement de N colonnes distinctes ou empilées (c'est-à-dire arrangées dans une enveloppe commune). Chaque colonne abrite un lit du milieu granulaire dans lequel s'écoule le fluide principal. Les N colonnes sont connectées entre elles par un réseau de conduites permettant éventuellement l'injection/mélange ou le soutirage d'un fluide secondaire, ces injections et soutirages possibles ayant lieu entre les colonnes. Les N colonnes fonctionnent en série au sens où le fluide sortant d'une colonne entre dans la colonne suivante.

La présente invention concerne un dispositif de distribution du fluide entrant dans une colonne de la série pour le répartir sur l'ensemble de la section du lit granulaire.

La présente invention concerne également un dispositif qui permet de collecter en fond de lit sur la section complète de la colonne le fluide principal pour l'amener dans la conduite inter-colonne qui permet de transférer ledit fluide vers la colonne suivante.

L'invention consiste essentiellement en un aménagement de la zone de distribution située en tête de colonne ou de collecte située en fond de colonne.

L'invention permet de compenser de façon avantageuse la distribution de temps de séjour du fluide dans une colonne entre les deux zones dites non sélectives, c'est-à-dire en dehors du milieu granulaire, que constituent la zone de distribution en tête de lit et la zone de collecte en fond de lit, tout en minimisant fortement les volumes des zones dites non sélectives.

En effet, l'invention concerne les procédés de séparation en Lit Mobile Simulé (LMS) pour lesquels la synchronicité de l'ensemble des particules de fluides au sein du lit, et au sein du réseau d'injection / collecte est particulièrement critique pour garantir les hauts niveaux de pureté et de rendement requis par le procédé. On parle dans la suite de manière abrégée de synchronicité du fluide.

On entend par synchronicité du fluide le fait que si l'on divise par la pensée la section de la colonne en une multiplicité de canaux fluides évoluant en parallèle, chaque canal fluide doit avoir autant que possible le même temps de séjour depuis son entrée dans la colonne jusqu'à sa sortie.

Par ailleurs, toute augmentation des volumes des zones non sélectives induit une augmentation non désirée des débits « de tourne en rond » (traduction de la terminologie anglo saxonne « pump around » ) dans l'installation, à mêmes niveaux de performance.

### DESCRIPTION SOMMAIRE DES FIGURES

L'ensemble des figures est représenté pour un écoulement descendant, mais peut concerner aussi, comme il sera expliqué plus loin également le cas d'un écoulement l'invention est strictement symétrique pour un écoulement ascendant.

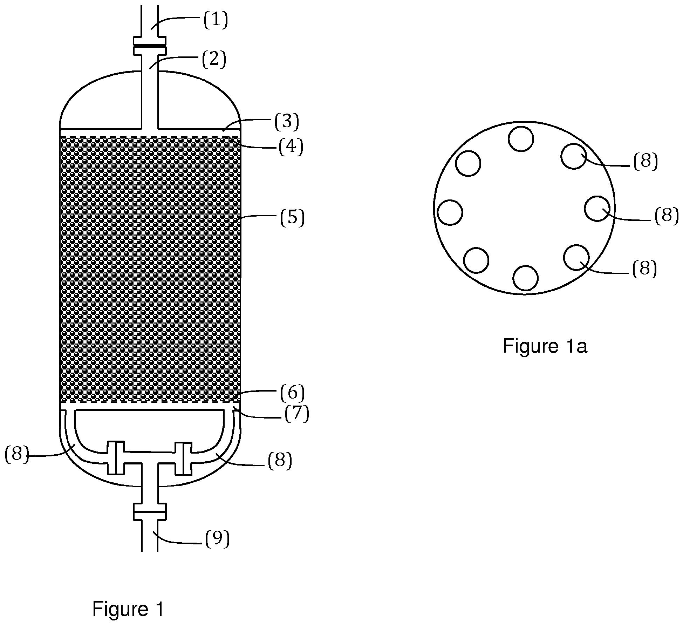

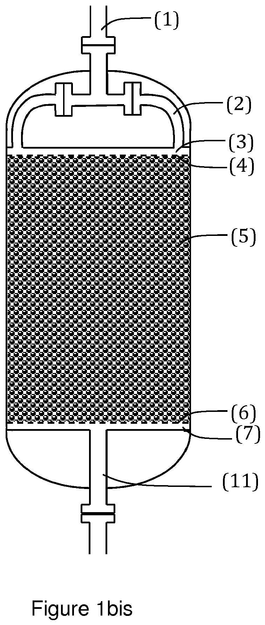

La figure 1 représente une vue schématique de l'ensemble de la colonne à écoulement axiale des fluides avec son dispositif de distribution des fluides en tête et son dispositif de collecte en fond.
La figure 1a est une vue de dessus qui permet de visualiser l'emplacement des conduites de collecte (8).
La figure 1 bis représente une variante de la colonne selon l'invention dans laquelle l'injection dans le lit se fait au moyen d'un réseau de conduites périphériques qui alimente le canal de distribution.
La figure 2 représente une vue schématique de la colonne dans une variante dite en écoulement radial du fluide avec le réseau de collecte selon l'invention.
La figure 2a est une vue de dessus qui permet de visualiser l'emplacement des conduites de collecte (8).

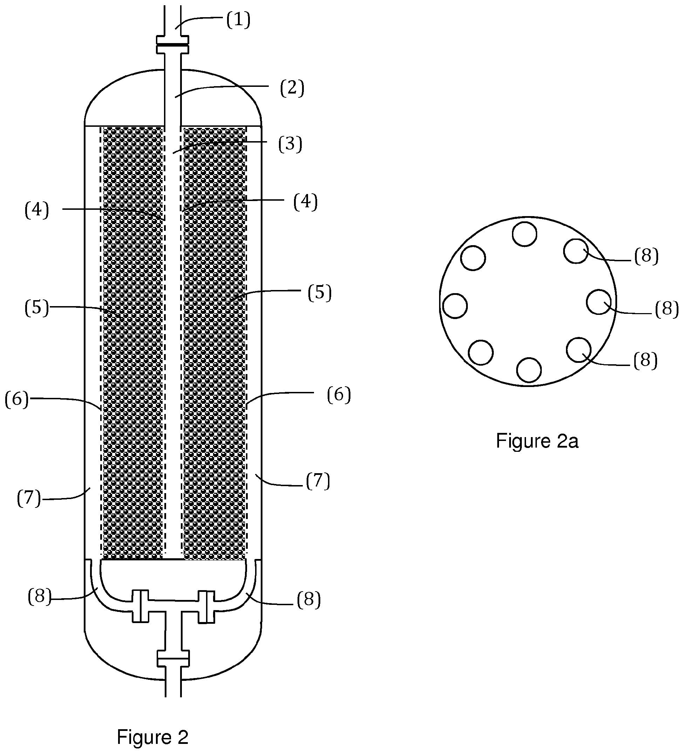

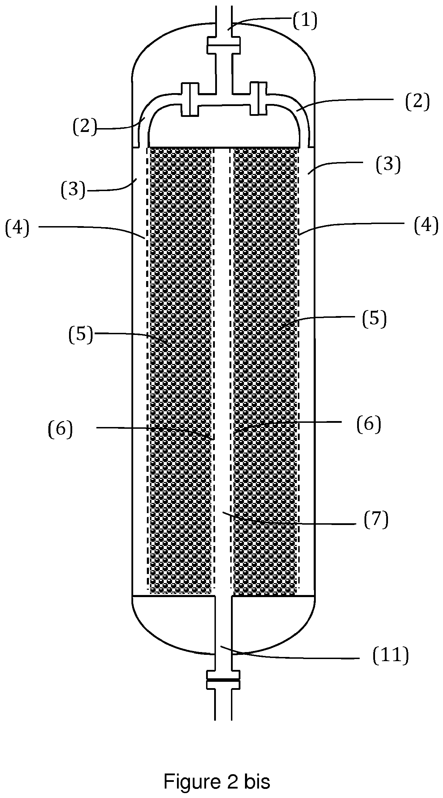

La figure 2 bis représente une vue schématique de la colonne dans une variante dite en écoulement radial du fluide avec le réseau d'alimentation selon l'invention.

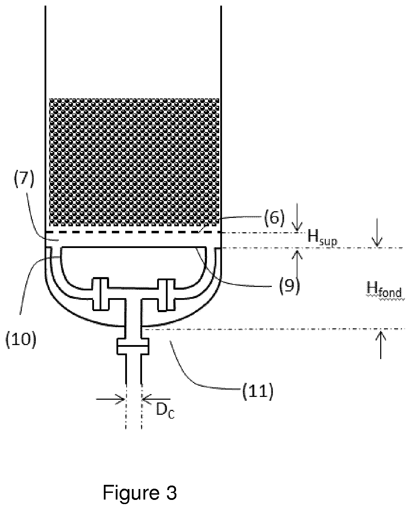

La figure 3 représente une vue plus détaillée du fond de la colonne suivant l'invention et montre les paramètres de dimensionnement Hsup, Hfond et le diamètre Dc de la conduite reliant une colonne à la suivante.

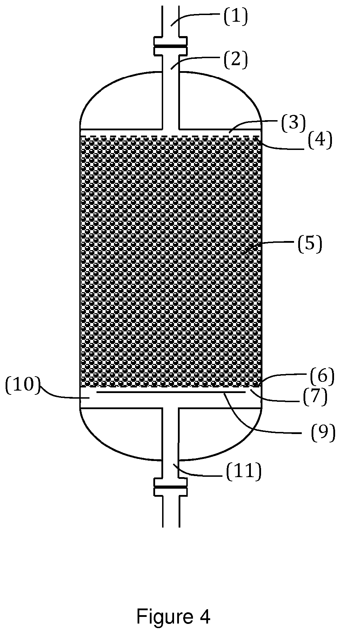

La figure 4, selon l'art antérieur, représente une vue schématique de l'ensemble de la colonne dans une configuration permettant de compenser les temps de séjour des zones non sélectives au moyen d'une chicane (9).

### EXAMEN DE L'ART ANTERIEUR

L'art antérieur enseigne comment dimensionner un distributeur pour une colonne multi étagée, c'est-à-dire constituée d'une multiplicité de plateaux disposés selon un axe sensiblement vertical, chaque plateau supportant un lit de solide granulaire.

Les brevets FR 2 794 663 A1, EP 0 842 687 A1, EP0074815, US2006/0108274A1, en particulier, fournissent des exemples de dispositifs de distribution/mélanges utilisés dans le cas de l'adsorption en lit mobile simulé pour une colonne multi-étagée. Ces dispositifs assurent de façon successive les fonctions de collecte du fluide principal en provenance du lit amont, de mélange du fluide principal avec le fluide secondaire, et de redistribution du mélange vers le lit aval.

Ces brevets décrivent la division de la section de la colonne en panneaux ou secteurs radiaux.

L'art antérieur enseigne également l'importance de garantir une bonne synchronicité dans le système de distribution pour obtenir les performances requises pour la séparation.

En particulier le brevet FR2933000 propose d'ajouter un élément de compensation du temps de séjour, une chicane, dans la zone non sélective juste avant ou juste après le plateau distributeur en entrée de colonne.

Le document de Silva M., Rodrigues A. et Mota J. « Effect of dead volumes on the performance of an industrial-scale simulated moving-bed parex unit or p-Xylene purification », paru dans l'AIChE Journal janv. 2016, vol 62, n°1, (et qu'on peut traduire par « effets des volumes morts sur les performances d'une unité Parex en lit mobile simulé »), enseigne l'importance de diminuer les volumes non sélectifs car l'augmentation de ces volumes entraine une baisse de pureté et une augmentation de la consommation de désorbant.

Une mise en œuvre d'un lit mobile simulé par un système à N-colonnes en série permet d'assurer les injections et collectes ainsi que les mélanges en un seul point entre deux colonnes dans le réseau. L'homme du métier sait donc qu'il peut ainsi s'affranchir des problèmes de rinçage, car il minimise ainsi les volumes non rincés par le débit de tourne en rond.

### DESCRIPTION SOMMAIRE DE L'INVENTION

La présente invention peut se définir comme un dispositif de distribution du fluide dans une colonne comportant un lit de solide granulaire, ce solide étant un solide adsorbant, ladite colonne faisant partie d'un ensemble de N colonnes fonctionnant en série. La valeur de N est généralement comprise entre 4 et 24, préférentiellement entre 6 et 15.

Ce dispositif est particulièrement adapté au procédé de séparation en lit mobile simulé, car il permet une réduction substantielle des volumes dit non sélectifs, comme cela est illustré dans l'exemple comparatif faisant partie de la présente demande.

Dans la suite on parle de fluide entrant pour désigner le fluide entrant dans la colonne et de fluide sortant pour désigner le fluide sortant de la colonne.

Le procédé utilisant le présent dispositif fait généralement appel à plusieurs colonnes en série, chaque colonne utilisant le dispositif selon l'invention, soit comme moyen d'introduction du fluide entrant, soit comme moyen de collecte du fluide sortant. C'est pourquoi dans le texte on parle d'un dispositif de distribution du fluide entrant ou de collecte du fluide sortant et quelquefois plus simplement d'un dispositif de distribution ou de collecte. Les colonnes constituant la série de N colonnes peuvent être à écoulement axial ou radial du fluide à l'intérieur du lit granulaire.

Toute série de N colonnes, chaque colonne étant munie du dispositif selon l'invention, soit comme moyen d'introduction du fluide entrant, soit comme moyen de collecte du fluide sortant rentre dans le cadre de la présente invention.

De façon plus précise le dispositif de distribution du fluide entrant, ou de collecte du fluide sortant, dans une colonne munie d'un lit de solide granulaire adsorbant, ladite colonne faisant partie d'une série de N colonnes disposées en série, peut se définir comme un dispositif dans lequel la distribution du fluide entrant au niveau d'une colonne est réalisée au moyen d'une pluralité de conduites périphériques (2) régulièrement réparties à partir d'une conduite d'alimentation unique sensiblement centrale, ou bien la collecte du fluide sortant est réalisée au moyen de conduites périphériques (8) régulièrement réparties sur la section de la colonne et se réunissant en une conduite unique sensiblement centrale. La vitesse débitante dans les canaux d'injection du fluide entrant, ou de collecte du fluide sortant, est comprises entre 1 et 6 m.s⁻¹, et le nombre de conduites périphériques (2) ou (8) varie entre 4 et 20, préférentiellement entre 6 et 12.

Dans la variante dans laquelle l'écoulement du fluide dans chaque colonne de la série se fait de façon axiale, les dimensions principales de chaque colonne sont les suivantes :
- un diamètre compris entre 1 et 15 m, préférentiellement entre 7 et 12 m,
- une hauteur du lit granulaire comprise entre 0,2 et 1,5 m, préférentiellement entre 0,4 et 1m.

Dans la variante dans laquelle l'écoulement du fluide dans chaque colonne se fait de façon radiale depuis la périphérie vers le centre, les dimensions principales de chaque colonne sont les suivantes :
- un diamètre entre 3 et 15 m,
- une hauteur permettant de développer une section comprise entre 1 et 200 m², préférentiellement entre 20 et 80 m², ladite section étant calculée comme celle du cylindre de hauteur la hauteur du lit granulaire et de rayon, tout rayon compris entre le rayon du collecteur d'entrée et le rayon du collecteur central,
- l'épaisseur du lit granulaire variant entre 0,2 et 1,5 m, et préférentiellement entre 0,4 et 1m.

De façon plus précise, la présente invention peut donc se définir comme un dispositif de distribution de fluide entrant ou de collecte du fluide sortant dans une ou plusieurs colonnes munies d'un lit de solide granulaire, dispositif dans lequel :
- la distribution du fluide est réalisée au moyen d'une pluralité de conduites périphériques régulièrement réparties à partir d'une conduite d'alimentation unique sensiblement centrale, le nombre de conduites périphériques variant entre 4 et 20, préférentiellement entre 6 et 12,
- ou bien la collecte du fluide sortant est réalisée au moyen de conduites périphériques régulièrement réparties sur la section de la colonne et se réunissant en une conduite unique sensiblement centrale, le nombre de conduites périphériques variant entre 4 et 20, préférentiellement entre 6 et 12.

Le présent dispositif s'applique particulièrement à un procédé de séparation en lit mobile simulé utilisant une pluralité de colonnes disposées en série. Le nombre de colonnes en série N variant de 4 à 24, et préférentiellement de 6 à 15.

Selon une première variante, présentée sur les figures 1 et 1a, le procédé en lit mobile simulé utilisant le dispositif de collecte selon l'invention peut se décrire de la façon suivante : La circulation du fluide à l'intérieur du lit de solide granulaire est axiale, et l'injection dans le lit se fait au moyen d'une conduite (2) sensiblement centrée sur l'axe vertical de la colonne, qui alimente le canal de distribution horizontal (3), le lit de solide granulaire (5) étant ensuite alimenté depuis ledit canal de distribution (3) au travers d'une grille (4), et le fluide s'écoulant au travers du lit granulaire (5) selon une direction sensiblement verticale, le fluide étant ensuite collecté sous la grille (6) à partir du canal de collecte (7) par l'intermédiaire des conduites périphériques (8), et l'ensemble du débit étant rassemblé dans une conduite d'évacuation unique (9) sensiblement centrée selon l'axe vertical de la colonne, la vitesse débitante du fluide sortant dans les conduites périphériques (8) étant comprise entre 1 et 6 m.s-1.

Selon une seconde variante, présentée sur la figure 1bis, le procédé en lit mobile simulé utilisant le dispositif de distribution selon l'invention peut se décrire de la façon suivante :
La circulation du fluide à l'intérieur du lit de solide granulaire est axiale, et l'introduction du fluide dans le lit se fait au moyen d'un réseau de conduites périphériques (2) qui alimente le canal de distribution (3), puis au travers d'une grille (4), le fluide s'écoulant à l'intérieur du lit granulaire (5) selon une direction sensiblement verticale, la collecte du fluide sortant se faisant à partir du canal de collecte (7) au moyen d'une conduite unique (11) sensiblement centrée sur l'axe vertical de la colonne, la vitesse débitante du fluide entrant dans les conduites périphériques (2) étant comprise entre 1 et 6 m.s-1.

Le sens de l'écoulement axial peut être vertical vers le haut ou vertical vers le bas. Le dispositif de collecte en écoulement vers le bas est alors le même que le dispositif de distribution en écoulement vers le haut.

Selon une troisième variante, présentée sur les figures 2 et 2a, le procédé utilisant le dispositif de distribution selon l'invention peut se décrire de la façon suivante :
La circulation des fluides à l'intérieur du lit de solide granulaire se fait de façon radiale, le fluide étant introduit au centre de la colonne par le canal central (2), puis traversant le lit granulaire (5) depuis le centre vers la périphérie où il est collecté dans la zone périphérique (7) qui ramène le liquide par l'intermédiaire des conduites périphériques (8) vers la conduite d'évacuation (9) sensiblement centrée selon l'axe de la colonne, la vitesse débitante du fluide sortant dans les conduites périphériques (8) étant comprise entre 1 et 6 m.s-1.

Selon une quatrième variante, présentée sur la figure 2bis, le procédé utilisant le dispositif de distribution selon l'invention peut se décrire de la façon suivante :
La circulation des fluides à l'intérieur du lit de solide granulaire se fait de façon radiale, le fluide étant introduit dans le lit granulaire depuis une conduite centrale (1) qui se divise en une pluralité de conduites périphériques (8) permettant d'alimenter le lit granulaire (5), le fluide traversant ledit lit depuis la périphérie vers le centre où il est collecté dans le canal central (7) puis est évacué par la conduite (11) sensiblement centrée sur l'axe vertical de la colonne, la vitesse débitante du fluide entrant dans les conduites périphériques (8) étant comprise entre 1 et 6 m.s-1.

Le procédé de séparation en lit mobile simulé faisant appel au dispositif selon l'invention peut être tel que la charge à séparer est un mélange quelconque de composés aromatiques ayant de 7 à 9 atomes de carbone.

Le procédé de séparation en lit mobile simulé faisant appel au dispositif selon l'invention peut être tel que la charge à séparer est un mélange de normales et d'iso paraffines.

Le procédé de séparation en lit mobile simulé faisant appel au dispositif selon l'invention peut être tel que la charge à séparer est un mélange de normales et d'iso oléfines.

Le procédé de séparation en lit mobile simulé faisant appel au dispositif selon l'invention peut être tel que le fluide principal traversant ledit dispositif a une masse volumique comprise entre 600 et 950 kg/m3 une viscosité comprise entre 0,1 et 0,6 cPo.

### DESCRIPTION DETAILLEE DE L'INVENTION

Le problème que cherche à résoudre la présente invention est celui de limiter les écarts de temps de séjour dans les zones non sélectives d'un système N-colonnes fonctionnant en série, écarts qui nuisent aux performances de la séparation, tout en minimisant les volumes desdites zones non sélectives (appelés plus simplement dans la suite volumes non sélectifs) qui induisent une augmentation non désirée du débit de « tourne en rond » de l'installation à mêmes niveaux de performance qu'un système dépourvu de zone non sélective.

L'invention porte sur un système qui permet de garantir une bonne synchronicité du fluide dans le système de distribution/collecte à l'échelle de la section complète de la colonne, et qui réalise par ailleurs un faible volume non sélectif en disposant de manière adéquate le réseau de conduite de distribution et de collecte. On entend par synchronicité le fait que chaque élément de fluide entrant a approximativement le même temps de séjour dans la colonne que les autre éléments entrant. C'est-à-dire que la distribution de temps de séjour est une courbe de Gauss avec un écart type le plus réduit possible. Dans la pratique, on estime qu'on a réalisé une synchronicité correcte si la dispersion des temps de séjour est à plus ou moins 10% par rapport à la valeur moyenne.

Les colonnes selon l'invention peuvent être organisées selon deux modes d'écoulement :
- Une mise en œuvre axiale dans laquelle l'écoulement au sein du lit granulaire se fait essentiellement suivant l'axe vertical de la colonne, de haut en bas ou de bas en haut.
- Une mise en œuvre radiale dans laquelle l'écoulement au sein du lit granulaire se fait essentiellement de la périphérie vers le centre de la colonne, ou du centre de la colonne vers la périphérie .

Les dimensions des colonnes sont les suivantes en fonction du mode d'écoulement:
- Pour une mise en œuvre axiale, un diamètre entre 1 et 15 m, préférentiellement entre 7 et 12 m. La hauteur du lit granulaire varie entre 0,2 et 1,5 m, préférentiellement entre 0,4 et 1m.
- Pour une mise en œuvre radiale, un diamètre entre 3 et 15 m, une hauteur permettant de développer une section comprise entre 1 et 200 m², préférentiellement entre 20 et 80 m². La section du lit granulaire se définit comme celle du cylindre de hauteur la hauteur du lit granulaire et de rayon, tout rayon compris entre le rayon du cylindre correspondant au collecteur d'entrée et le rayon du cylindre correspondant au collecteur central. La section du lit granulaire évolue donc en fonction du rayon depuis le collecteur d'entrée jusqu'au collecteur de sortie. L'épaisseur du lit granulaire varie entre 0,2 et 1,5 m, préférentiellement entre 0,4 et 1m.

Les injections et collectes ainsi que les mélanges sont faits en un seul point entre deux colonnes dans le réseau (1).

Selon un premier mode de réalisation représenté sur la figure 1, la mise en œuvre du lit est axiale et l'injection dans le lit se fait au moyen d'une conduite (2) sensiblement centrée sur l'axe vertical de la colonne, qui alimente par un jet le canal de distribution horizontal (3).

Le lit granulaire (5) est ensuite alimenté depuis le canal de distribution (3) au travers d'une grille (4).

Le fluide s'écoule au travers du lit granulaire (5) selon une direction sensiblement verticale.

Le fluide est ensuite collecté sous la grille (6) dans le canal de collecte (7).

L'ensemble du débit est rassemblé par le réseau de conduites de collecte périphérique (8).

On parle de collecte périphérique car les points de récupération (8) du fluide sortant à partir du canal de collecte (7) sont en périphérie dudit canal, comme montré sur la figure 1 a en vue de dessus.

Selon un second mode de réalisation, représentée par la figure 1 bis, la mise en œuvre du lit est axiale et l'injection dans le lit se fait au moyen d'un réseau de conduites périphériques qui alimente le canal de distribution (3). La collecte se fait au moyen d'une conduite unique (11) centrée dans la colonne qui collecte le débit depuis le canal de collecte.

L'enchainement des colonnes peut se faire de deux manières :
Soit les colonnes sont toutes avec un système de distribution selon l'invention, soit elles sont toutes avec un système de collecte selon l'invention. On peut aussi enchainer les colonnes de façon alternée, c'est-à-dire une colonne ayant un système de collecte selon l'invention, et la suivante ayant une système de distribution selon l'invention. On reste parfaitement dans le cadre de la présente invention en enchainant les colonnes munies d'un système de distribution ou de collecte selon l'invention.

Les hauteurs des canaux d'injection et de collecte sont dimensionnées telles qu'en aucun point la vitesse débitante n'excède une vitesse de dimensionnement comprise entre 1 et 8 m/s.

Le nombre de conduites variera entre 4 et 20, préférentiellement entre 6 et 12. Les sections de conduite seront dimensionnées pour assurer une vitesse des fluides comprise entre 1 et 8 m/s.

Selon un troisième mode de réalisation, représenté par la figure 2, la mise en œuvre du lit est radiale et l'injection dans le lit se fait au moyen d'une conduite verticale (2), ou d'un tube annulaire, positionné sensiblement au centre de la colonne. Le liquide s'écoule radialement dans le lit (5) depuis le centre vers la périphérie du lit, et est collecté dans les zones périphériques (7). L'ensemble du débit est collecté par le réseau de conduites (8) situé en périphérie qui ramène le fluide sortant dans la conduite d'évacuation (9).

Selon un quatrième mode de réalisation représenté par la figure 2 bis, la mise en œuvre du lit est radiale et l'injection dans le lit se fait au moyen d'un réseau de conduites périphériques. Le liquide s'écoule radialement dans le lit (5) depuis la périphérie vers le centre de la colonne, et est collecté dans le canal central (7). L'ensemble du débit est collecté par une conduite creuse ou un tube annulaire (11) positionné sensiblement au centre de la colonne et récupérant le fluide sortant issu du canal central (7).

### EXEMPLES SELON L'ART ANTERIEUR ET SELON L'INVENTION

L'objectif de cet exemple comparatif est de fournir les valeurs de volumes non sélectifs et de dispersion dans 3 cas :
a) l'art antérieur sans compensation des temps de séjour
b) l'art antérieur avec compensation des temps de séjour au moyen d'une chicane (9) telle que représentée sur la figure 4,
c) la présente invention.

On considère une colonne de 2 m de diamètre comportant un lit granulaire de 1 m de haut. L'écoulement d'un fluide de densité 725 kg/m³ et de viscosité 0,2 cP se fait à l'intérieur de la colonne avec une vitesse superficielle de 2 cm/s. Cette vitesse superficielle est calculée sur la section de la colonne supposée libre.

Pour dimensionner cette colonne sans compensation de temps de séjour, suivant le schéma de la figure 4 sans la chicane 9 sur la figure 4 (qui correspond à la solution de l'art antérieur), l'homme de l'art se fixera une vitesse maximale dans les canaux de distribution et de collecte et dans les conduites de distributions et de collecte, cette vitesse servira à dimensionner lesdits canaux et lesdites conduites. Dans cet exemple, une vitesse maximale de 5 m/s est utilisée. Les hauteurs des canaux en résultant sont de 3,2 cm, le diamètre des conduites de 12,6 cm.
a) Il en résulte, pour un dimensionnement sans compensation de temps de séjour, un volume non sélectif (distribution et collecte) de 0,21 m³ et une dispersion de 9,4 s².

L'art antérieur présenté dans le brevet FR2933000 explique comment dimensionner une chicane pour redresser la distribution des temps de séjour. Le ratio entre la section annulaire (passage du liquide entre le bord la chicane et la paroi de la colonne) et la section totale de la colonne est choisi identique à celui de l'exemple du brevet, soit 8,3 %. La chicane (9) a donc un diamètre L_{c} de 1,91 m. Comme présenté sur la figure 4, avec une chicane, il y a un canal de collecte supérieure (7) et un canal inférieur (10). Les hauteurs requises sont de 0,21 cm pour le canal supérieur et de 3,2 cm pour le canal inférieur.

b) Pour un dimensionnement avec compensation de séjour au moyen d'une chicane (9) suivant l'art antérieur, le volume non sélectif (distribution et collecte) est de 0,22 m³ et la dispersion de 3,1 s².

c) Suivant la présente invention, nous retenons pour le canal supérieur le même critère de dimensionnement que dans l'art antérieur. Le diamètre des 8 conduites de collecte périphériques (8), dimensionnées sur le critère d'une vitesse maximale de 5 m.s⁻¹, est de 4,5 cm.

Il en résulte, pour un dimensionnement suivant l'invention, un volume non sélectif (distribution et collecte) de 0,13 m³ et une dispersion de 3,2 s².

La présente invention permet donc un gain de 40% sur le volume non sélectif, tout en maintenant un gain notable sur la dispersion de temps de séjour. Or la réduction du volume non sélectif, à dispersion équivalente, est un élément essentiel dans le maintien des performances de procédé de séparation en lit mobile simulé.

| | Volume des zones non sélectives (m³) | Dispersion des zones non sélectives (s²) |
|---|---|---|
| a)Dimensionnement suivant l'art antérieur sans compensation | 0,21 | 9,4 |
| b)Dimensionnement suivant l'art antérieur avec compensation | 0,22 | 3,1 |
| c)Dimensionnement suivant l'invention | 0,13 | 3,2 |

## Revendications

1. Colonne à lit de solide granulaire adsorbant et adaptée pour faire partie d'une série de N colonnes disposées en série, le nombre de colonnes N en série étant compris entre 4 et 24, et préférentiellement compris entre 6 et 15,
la colonne comprenant une pluralité de conduites périphériques (2) régulièrement réparties à la périphérie de la colonne, reliées à une conduite d'alimentation unique (1) sensiblement centrale, chaque conduite périphérique (2) alimentant un canal de distribution (3) de la colonne, suivi en aval selon le sens d'écoulement du fluide, d'une grille (4), la collecte du fluide sortant de la colonne se faisant à partir d'un canal de collecte (7) au moyen d'une conduite unique (11) sensiblement centrée sur l'axe vertical de la colonne, la vitesse débitante du fluide entrant dans les conduites périphériques (2) étant comprise entre 1 et 6 m.s-1, et l'écoulement du fluide dans la colonne étant axial ou radial, et le nombre de conduites périphériques (2) variant entre 4 et 20, préférentiellement entre 6 et 12, ou
la colonne comprenant une conduite centrale (2) sensiblement centrée sur l'axe vertical de la colonne, reliée à une conduite d'alimentation unique (1) sensiblement centrale, la conduite centrale (2) alimentant un canal de distribution (3) de la colonne, suivi en aval selon le sens d'écoulement du fluide, d'une grille (4), la collecte du fluide sortant de la colonne se faisant à partir d'un canal de collecte (7) par l'intermédiaire d'une pluralité de conduites périphériques (8) régulièrement réparties à la périphérie de la colonne, et l'ensemble du débit étant rassemblé dans une conduite d'évacuation unique (9) sensiblement centrée selon l'axe vertical de la colonne, la vitesse débitante du fluide sortant des conduites périphériques (8) étant comprise entre 1 et 6 m.s-1, et l'écoulement du fluide dans la colonne étant axial ou radial, et le nombre de conduites périphériques (8) variant entre 4 et 20, préférentiellement entre 6 et 12.

2. Colonne à lit de solide granulaire adsorbant selon la revendication 1, dans laquelle pour un écoulement axial du fluide à l'intérieur de la colonne, les dimensions principales de la colonne sont les suivantes :
- un diamètre compris entre 1 et 15 m, préférentiellement entre 7 et 12 m,
- une hauteur du lit granulaire comprise entre 0,2 et 1,5 m, préférentiellement entre 0,4 et 1m.

3. Colonne à lit de solide granulaire adsorbant selon la revendication 1, dans laquelle pour un écoulement radial du fluide à l'intérieur de la colonne, les dimensions principales de la colonne sont les suivantes :
- un diamètre entre 3 et 15 m,
- une hauteur permettant de développer une section comprise entre 1 et 200 m², préférentiellement entre 20 et 80 m², ladite section étant calculée comme celle du cylindre de hauteur la hauteur du lit granulaire et de rayon, tout rayon compris entre le rayon du collecteur d'entrée et le rayon du collecteur central,
- l'épaisseur du lit granulaire variant entre 0,2 et 1,5 m, et préférentiellement entre 0,4 et 1m.

4. Procédé de séparation en lit mobile simulé utilisant la colonne à lit de solide granulaire adsorbant selon l'une quelconque des revendications 1 à 3, dans lequel la charge à séparer est un mélange quelconque de composés aromatiques ayant de 7 à 9 atomes de carbone.

5. Procédé de séparation en lit mobile simulé utilisant la colonne à lit de solide granulaire adsorbant selon l'une quelconque des revendications 1 à 3, dans lequel la charge à séparer est un mélange de normales et d'iso paraffines.

6. Procédé de séparation en lit mobile simulé utilisant la colonne à lit de solide granulaire adsorbant selon l'une quelconque des revendications 1 à 3, dans lequel la charge à séparer est un mélange de normales et d'iso oléfines.

7. Procédé de séparation en lit mobile simulé utilisant la colonne à lit de solide granulaire adsorbant selon l'une quelconque des revendications 1 à 3, dans lequel le fluide principal traversant ledit dispositif a une masse volumique comprise entre 600 et 950 kg/m3 une viscosité comprise entre 0,1 et 0,6 cP.

## Patentansprüche

1. Säule mit Bett aus adsorbierendem körnigem Feststoff, welche dafür eingerichtet ist, Teil einer Reihe von N in Reihe angeordneten Säulen zu sein, wobei die Anzahl N der Säulen in Reihe zwischen 4 und 24 und vorzugsweise zwischen 6 und 15 beträgt,
wobei die Säule mehrere gleichmäßig am Umfang der Säule verteilte periphere Leitungen (2) umfasst, die mit einer einzigen, im Wesentlichen zentralen Speiseleitung (1) verbunden sind, wobei jede periphere Leitung (2) einen Verteilungskanal (3) der Säule speist, dem stromabwärts, auf die Strömungsrichtung des Fluids bezogen, ein Gitter (4) folgt, wobei das Sammeln des aus der Säule ausströmenden Fluids aus einem Sammelkanal (7) mittels einer einzigen, im Wesentlichen auf der vertikalen Achse der Säule zentrierten Leitung (11) erfolgt, wobei die Durchflussgeschwindigkeit des in die peripheren Leitungen (2) einströmenden Fluids zwischen 1 und 6 m.s⁻¹ liegt und die Strömung des Fluids in der Säule axial oder radial verläuft und die Anzahl der peripheren Leitungen (2) zwischen 4 und 20, vorzugsweise zwischen 6 und 12 variiert, oder
wobei die Säule eine im Wesentlichen auf der vertikalen Achse der Säule zentrierte zentrale Leitung (2) umfasst, die mit einer einzigen, im Wesentlichen zentralen Speiseleitung (1) verbunden ist, wobei die zentrale Leitung (2) einen Verteilungskanal (3) der Säule speist, dem stromabwärts, auf die Strömungsrichtung des Fluids bezogen, ein Gitter (4) folgt, wobei das Sammeln des aus der Säule ausströmenden Fluids aus einem Sammelkanal (7) über mehrere gleichmäßig am Umfang der Säule verteilte periphere Leitungen (8) erfolgt und der gesamte Durchflussstrom in einer einzigen, im Wesentlichen entlang der vertikalen Achse der Säule zentrierten Abflussleitung (9) gesammelt wird, wobei die Durchflussgeschwindigkeit des aus den peripheren Leitungen (8) ausströmenden Fluids zwischen 1 und 6 m.s⁻¹ liegt und die Strömung des Fluids in der Säule axial oder radial verläuft und die Anzahl der peripheren Leitungen (8) zwischen 4 und 20, vorzugsweise zwischen 6 und 12 variiert.

2. Säule mit Bett aus adsorbierendem körnigem Feststoff nach Anspruch 1, wobei für eine axiale Strömung des Fluids im Inneren der Säule die Hauptabmessungen der Säule die folgenden sind:
- ein Durchmesser zwischen 1 und 15 m, vorzugsweise zwischen 7 und 12 m,
- eine Höhe des körnigen Betts zwischen 0,2 und 1,5 m, vorzugsweise zwischen 0,4 und 1 m.

3. Säule mit Bett aus adsorbierendem körnigem Feststoff nach Anspruch 1, wobei für eine radiale Strömung des Fluids im Inneren der Säule die Hauptabmessungen der Säule die folgenden sind:
- ein Durchmesser zwischen 3 und 15 m,
- eine Höhe, die es ermöglicht, einen Querschnitt zwischen 1 und 200 m², vorzugsweise zwischen 20 und 80 m² zu entwickeln, wobei dieser Querschnitt berechnet wird als derjenige des Zylinders mit einer Höhe, die gleich der Höhe des körnigen Betts ist, und einem Radius, der gleich einem beliebigen Radius zwischen dem Radius des Eintrittssammlers und dem Radius des zentralen Sammlers ist,
- wobei die Dicke des körnigen Betts zwischen 0,2 und 1,5 m und vorzugsweise zwischen 0,4 und 1 m variiert.

4. Trennverfahren in einem simulierten Wanderbett unter Verwendung der Säule mit Bett aus adsorbierendem körnigem Feststoff nach einem der Ansprüche 1 bis 3, wobei die zu trennende Charge eine beliebige Mischung aromatischer Verbindungen mit 7 bis 9 Kohlenstoffatomen ist.

5. Trennverfahren in einem simulierten Wanderbett unter Verwendung der Säule mit Bett aus adsorbierendem körnigem Feststoff nach einem der Ansprüche 1 bis 3, wobei die zu trennende Charge eine Mischung aus normalen Paraffinen und Isoparaffinen ist.

6. Trennverfahren in einem simulierten Wanderbett unter Verwendung der Säule mit Bett aus adsorbierendem körnigem Feststoff nach einem der Ansprüche 1 bis 3, wobei die zu trennende Charge eine Mischung aus normalen Olefinen und Isoolefinen ist.

7. Trennverfahren in einem simulierten Wanderbett unter Verwendung der Säule mit Bett aus adsorbierendem körnigem Feststoff nach einem der Ansprüche 1 bis 3, wobei das Hauptfluid, das die Vorrichtung durchströmt, eine volumenbezogene Masse zwischen 600 und 950 kg/m³ und eine Viskosität zwischen 0,1 und 0,6 cP aufweist.

## Claims

1. Column with adsorbent granular solid bed adapted to form part of a series of N columns disposed in series, the number of columns N in series of between 4 and 24, and preferentially of between 6 and 15,
the column comprising a plurality of peripheral ducts (2) evenly distributed at the periphery of the column, linked to a single, substantially central supply duct (1), each peripheral duct (2) supplying a distribution channel (3) of the column, followed downstream in the direction of flow of the fluid, by a grating (4), the collection of the fluid leaving the column being done from a collection channel (7) by means of a single duct (11) substantially centred on the vertical axis of the column, the discharge velocity of the fluid entering into the peripheral ducts (2) being between 1 and 6 m.s⁻¹, and the flow of the fluid in the column being axial or radial, and the number of peripheral ducts (2) varying between 4 and 20, preferentially between 6 and 12, or
the column comprising a central duct (2) substantially centred on the vertical axis of the column, linked to a single, substantially central supply duct (1), the central duct (2) supplying a distribution channel (3) of the column, followed downstream in the direction of flow of the fluid, by a grating (4), the collection of the fluid leaving the column being done from a collection channel (7) via a plurality of peripheral ducts (8) evenly distributed at the periphery of the column, and all throughput being gathered in a single discharge duct (9) substantially centred on the vertical axis of the column, the discharge velocity of the fluid leaving the peripheral ducts (8) being between 1 and 6 m.s⁻¹, and the flow of the fluid in the column being axial or radial, and the number of peripheral ducts (8) varying between 4 and 20, preferentially between 6 and 12.

2. Column with adsorbent granular solid bed according to Claim 1, wherein, for an axial flow of the fluid inside the column, the main dimensions of the column are as follows:
- a diameter of between 1 and 15 m, preferentially between 7 and 12 m,
- a height of the granular bed of between 0.2 and 1.5 m, preferentially between 0.4 and 1 m.

3. Column with adsorbent granular solid bed according to Claim 1, wherein, for a radial flow of the fluid inside the column, the main dimensions of the column are as follows:
- a diameter between 3 and 15 m,
- a height allowing a section to be developed of between 1 and 200 m², preferentially between 20 and 80 m², said section being calculated as that of the cylinder whose height is the height of the granular bed and whose radius is any radius of between the radius of the inlet header and the radius of the central collector,
- the thickness of the granular bed varying between 0.2 and 1.5 m, and preferentially between 0.4 and 1 m.

4. Simulated moving bed separation method using the column with adsorbent granular solid bed according to any one of Claims 1 to 3, wherein the charge to be separated is any mixture of aromatic compounds having from 7 to 9 carbon atoms.

5. Simulated moving bed separation method using the column with adsorbent granular solid bed according to any one of Claims 1 to 3, wherein the charge to be separated is a mixture of normals and of iso-paraffins.

6. Simulated moving bed separation method using the column with adsorbent granular solid bed according to any one of Claims 1 to 3, wherein the charge to be separated is a mixture of normals and of iso-olefins.

7. Simulated moving bed separation method using the column with adsorbent granular solid bed according to any one of Claims 1 to 3, wherein the main fluid passing through said device has a density of between 600 and 950 kg/m3 and a viscosity of between 0.1 and 0.6 cP.
